# EUROPEAN PATENT SPECIFICATION

(11) **EP 2 948 881 B1**
(45) Date of publication and mention of the grant of the patent: **12.05.2021**
(21) Application number: 14710954.0
(22) Date of filing: 28.01.2014
(51) Int. Cl.: G16H 20/10, G16H 20/60

(54) **METHOD AND SYSTEM FOR A QUANTITATIVE SETTING OF THE INSULIN BOLUS FOR A DIABETIC PATIENT, AND FOR TIMING ITS ADMINISTRATION**
VERFAHREN UND SYSTEM ZUM QUANTITATIVEN EINSTELLEN DES INSULINBOLUS FÜR DIABETESPATIENTEN UND ZUR ZEITLICHEN STEUERUNG VON DESSEN VERABREICHUNG
PROCÉDÉ ET SYSTÈME DE DOSAGE QUANTITATIF DU BOLUS D'INSULINE POUR UN PATIENT DIABÉTIQUE, ET POUR RYTHMER SON ADMINISTRATION

(30) Priority: 28.01.2013 IT BO20130034
(43) Date of publication of application: 02.12.2015
(73) Proprietor: Meteda International SA, 6901 Lugano (CH)
(72) Inventor: VESPASIANI, Giacomo, 63039 San Benedetto del Tronto (AP) (IT)
(74) Representative: Ruzzu, Giammario
(86) International application number: PCT/IB2014/000084
(87) International publication number: WO 2014/115025

(56) References cited:
- EP-A1- 1 677 226
- WO-A1-2008/048588
- WO-A2-2008/030347
- US-A1- 2006 272 652
- US-A1- 2009 177 147

## Description

### Technical Field

The present invention relates to the technical field concerning telemedicine applied in order to help diabetic patients to correctly apply a therapeutic prescription.

In particular, the invention concerns a method and system for the definition of the insulin bolus, that is the insulin dose the patient must take in order to metabolize the amount of carbohydrates (CHOs) eaten during a meal, and for optimizing its distribution over time.

### Background Art

A correct handling of the diet and the dosage, as precise as possible, of the insulin bolus to be taken at each meal consumed by the patient, have always been considered cornerstones of success of the long-term diabetes treatment.

In this context, it is extremely important to make an estimate of the carbohydrates (CHOs) in grams contained in the food the patient is going to eat. The related insulin bolus is consequently computed on the basis of dosage tables defined by the specialist who is treating the patient and who uses the computing algorithms already well settled in the clinical medicine.

According to a simplified and exemplifying version of one of such algorithms, a personalized formula for computing the insulin dose for the bolus of one meal can be expressed as UI = gCHO x I/CHO, in which:
1. - UI are the insulin units to be taken for the meal bolus;
1. - gCHO is the CHO gram content in 100 grams of raw food, without rejects, multiplied by hectograms of the eaten food;
1. - I/CHO indicates the amount of carbohydrates which are metabolized by one insulin unit in the given person.

The modes in which the patient applies the specialist's indications relating to the personalized insulin dosage have been evolved over time, along with the evolution of the portable analysis instruments, of personal telecommunication terminals and telemedicine techniques.

Up to some years ago, it was the specialist who defined an optimal quantity of CHOs that the diabetic patient had to eat at each meal, and the corresponding insulin bolus to be injected before the meal. The patient was instructed as to the possible combinations and quantities of various foods composing the meal, necessary to reach the prefixed CHO content. Each time, the evaluation of the foods composition was let to the patient's ability and willingness. The blood glycemic level was controlled occasionally in out-patient departments or in analysis laboratories. In any case, there was no direct and continuous control of the results of the diabetes specialist prescription nor of the patient's actual application of the prescription. On the other hand, this task was difficult to perform for many patients. The evaluation of the food composition before each meal was complicated, often carried out in a rough way and, especially in the long run, occasionally.

The diffusion of the glucometers which were easy to use and available at reasonable price, and particularly of the interactive and programmable telephone terminals *(smartphones),*has made patients change their approach to the control of their blood glucose, and the diabetes specialist change their approach to the interaction with the patient.

The European Patent Application EP 1 677 226, filed by Vespasiani Giacomo, discloses a personal instrument for managing the meals composition and the dosing of the corresponding insulin bolus. The instrument is available on a smartphone and is assisted by the blood glucose analysis performed by a portable glucometer. There are also provided periodical interactions with the patient so as to get information about his style of life, such as regular physical activity or nutritional habits, and about special events regarding his state of health.

The instrument suggests the insulin bolus to inject before the meal based on the above information, suitably processed, and following the diabetes specialist's instructions as to insulin units for CHO units that the patient is going to eat in a given meal.

Moreover, the instrument helps the patient, in a simple and intuitive way, to quantify the foods that compose the menu of a given meal, by proposing directly the images of the portions of a given food and basing the calculation of the CHO content and of the relative insulin bolus on the actually chosen portions of the food.

The International Patent Application WO 2010/004406, filed by B.G. Informatica S.r.l., discloses a method and system for interactive management of the blood glucose control in the diabetic patient, which also uses a portable glucometer and a smartphone, preferably connected to each other, so as to compute and suggest to the patient the insulin bolus suitable to metabolize the carbohydrates contained in the meal he is going to eat.

The aforesaid application describes also a system of direct communication with the diabetes specialist, by means of a mobile telephone or Internet, in order to control the data acquired from the patient and the obtained results relating to the blood glucose control. As a result, the system allows the diabetes specialist to control the patient's situation in a substantially continuous way. Moreover, it allows the specialist to modify, if necessary, the prescription related to the insulin dosage for the given patient and the computing parameters present in the local algorithm adapted to define the insulin bolus based on the carbohydrates that the patient is going to eat in a meal, and the structure of the algorithm itself. All this occurs in a way fully transparent to the patient.

The international patent application WO 2008/0848588 discloses a method for calculating a meal bolus in an insulin pump. The insulin pump includes a pump mechanism and a memory storing food entries in a food database wherein each food entry is associated with a carbohydrate value. A programmable circuit is programmed to prompt selection of food entries and to calculate a meal bolus based on the selected food entries.

The above described interactive management systems, together with others, made available in the meantime, represent a valid aid for stabilizing the glucose blood level and avoiding, or at least delaying, the collateral effects of diabetes in the long run, that is, first of all the microvascular, macrovascular and cardiac complications.

### Disclosure of Invention

### Technical Problem

On the other hand, it is almost generally demonstrated that the precise evaluation of the carbohydrate part of the meals that the diabetic patient eats, is the most advanced instrument for the treatment of the diabetes, and especially of the type 1 diabetes. Its automation, and consequently, the fact that the quantitative computing of the CHOs actually consumed during a meal is no longer evaluated exclusively empirically by the patient, is a fundamental aspect of the optimization of the insulin treatment, and therefore, an important target in the improvement of the diabetic patient's life quality.

Essentially, using the conventional techniques, even the most modern, available so far, the diabetic patient is still asked to evaluate, before eating the meal, the type and the amount of the foods he will eat in the meal, and to take in advance the insulin bolus computed so as to metabolize the amount of CHOs present in the selected foods.

In this way, however, the amount of CHOs consumed for each meal, is in any case evaluated as a whole *a priori.* The patient is not sure that, once he has injected the calculated insulin dose, he will actually eat what he has planned. The course of the meal can be influenced by various factors, from a sudden loss of appetite to unexpected factors that can force the patient to interrupt the meal before its end, or to eat more than he has evaluated, etc.

Thus, the evaluation made before the meal must be inevitably imprecise and approximate.

On the other hand, in the physiology, the pancreas produces insulin each time a food is eaten, in particular if this food contains CHO. Consequently, the endogenous insulin is produced continuously and its level in the blood is adjusted practically in real time so as to metabolize the actually consumed CHOs. Therefore, it is obvious that taking the insulin bolus, no matter how carefully evaluated, computed for the given meal before that meal, is a rather rough imitation of the physiological process that leads to the CHO metabolization.

Moreover, it is necessary to take into consideration another aspect of the physiological process of the CHO metabolization. It is true that the major part of the insulin produced by the pancreas comes from the CHOs consumed directly by a subject. However, a smaller percentage of insulin is produced in order to metabolize the CHOs coming from the transformation of lipids and proteins consumed in the meantime.

Therefore, a correct evaluation of the insulin dose to administer to the diabetic patient should duly take into account the part introduced by the lipids and proteins contained in the portions of food consumed during the meal.

Furthermore, the lipids and proteins tend to rise the blood glucose according to a curve delayed in time with respect to the one produced by the directly consumed CHOs. The corresponding insulin should therefore be administered later with respect to the insulin necessary to metabolize the 'primitive' CHOs.

All the above makes it really very complicated to handle the insulin treatment with the simple use of the instruments available until now, and substantially impossible for a common, or even quite advanced diabetic patient.

### Technical Solution

An object of the present invention is to propose a method and a system for optimizing the distribution over time of the communication and information related to the administration of the insulin bolus necessary to metabolize the CHOs the diabetic patient has actually consumed during a meal, or in any case, for making the insulin administration as similar as possible to the physiological processes of insulin endogenous secretion in correspondence to a meal.

Another object of the invention is to propose a method and system capable of improving the accuracy and reliability of the insulin bolus calculated with respect to the CHOs actually consumed by the patient.

A further object of the invention is to propose a method and system capable of modifying the definition of the computation of of the insulin bolus and of its time distribution, so as to take into consideration the percentage of CHOs coming from the consumption of lipids and proteins.

The above mentioned objects are fully obtained by a method and a system for the quantitative definition of the insulin bolus for a diabetic patient and for the distribution overtime of its administration according to claims 1 and 7 respectively.

### Best Mode

The characteristics of the invention, as it will appear from the claims, are pointed out in the following detailed description.

According to the present invention, a diabetic patient who follows a self-monitoring insulin treatment advantageously has an interactive electronic terminal, for example, an interactive cellular telephone or smartphone, the use of which is extremely widespread nowadays. The patient has also a portable glucometer of the commercially available type, aimed at measuring the blood glucose in a simple way, in short time and at a reasonable price. In recent years, also the latter device has become of common use for the diabetic patient, by virtue of the gradual reduction of its cost as well as of its dimensions.

The diabetes specialist instructs the patient to periodically measure his blood glucose and to register the measured values, which is indispensable as a feedback of the insulin treatment.

Preferably, the patient has also means for the automatic and/or semi automatic insulin administration, for example, a microinfuser with a needle permanently installed subcutaneously. The microinfuser can continuously perform the basal insulin infusion, and moreover, it can be operated by the patient himself, generally before the meal, to inject an insulin bolus predetermined for each operation cycle. This device is particularly useful for optimizing the administered insulin dosage, for simplifying the administration operations and making them more rapid and less risky, and has allowed the diabetic patient to reduce his daily job related to the pathology management. For these reasons, the use of the microinfuser has gradually increased and at present, it is one of the most common supports of the diabetes mellitus treatment.

An insulin treatment management program runs on the patient's smartphone. Some of the program functions are already known, as they are common to various management programs of self-monitoring treatment, for example, the ones that carry out the inventions described in the above mentioned Patent Applications EP 1 677 226 and WO 2010/004406, to which reference is made.

In accordance with one of these functions, the patient periodically stores the values of some of his physiological parameters, such as the blood glucose values measured with the portable glucometer, in a permanent memory area of his smartphone. The values can be stored manually or by connecting the glucometer directly to the smartphone, by means of a Bluetooth or USB interface or in another standard communication way. A corresponding communication procedure of the management program interacts with the patient to allow him to carry out the above operation.

Another known function of the management program includes the interactive evaluation of the meal the patient is going to eat. The above function, performed by an interactive choice procedure of the program, is activated by the patient when he chooses various foods he is going to eat for his meal.

The choice procedure receives progressively at the input the information related to the type and quantity of the chosen food. Optionally, the procedure provides a further function of visual help to the choice, which presents to the patient the image of a portion of the chosen food, so as to allow him to verify its correspondence to what he is going to eat.

Once the interactive evaluation step is completed, the management program operates the automatic computing procedure which computes the quantity of carbohydrates (CHOs) contained in each of the different chosen foods, and the subsequent computing of the insulin bolus necessary to metabolize them.

The computing is performed on the basis of an algorithm whose code is stored in a program and data memory area of the smartphone. The algorithm structure is defined by the diabetes specialist who is treating the patient, on the basis of his professional evaluation of the situation and of the patient's therapeutic needs. The algorithm can duly take into consideration some physiological parameters, such as one or more values of blood glucose measured previously in self-monitoring and stored by the patient, or other parameters useful to evaluate the patient's present physical state.

The management program can also include a further procedure of updating the above mentioned algorithm and/or its computing parameters. The procedure is accessible only for the specialist, via a direct access during a visit at the doctor's office, or in the already described way, by means of a remote access directly from the specialist's office, on one of the communication lines available for the smartphone, for example, by means of the messages exchange on the cellular telephone network, or by means of an Internet connection.

After having computed the insulin bolus for a given meal, a conventional management program of the insulin treatment activates another communication procedure with the patient, which informs visually the patient about the ready units of insulin he must self-administer before the meal.

According to the invention, the method for the definition of the insulin bolus and for the distribution over time of its administration, carried out by a management program of the insulin treatment installed on a smartphone or another interactive electronic terminal (for example, a dedicated terminal), activated by the patient before a meal, includes first of all carrying out of a procedure for interactive evaluation of the type and quantity of each food.

The quantitative evaluation step for each food is activated in the way already described above. According to the method of the invention, directly after the deinition of each food, a computing step is carried out, by the activation of a corresponding procedure of the management program, for the computation of the CHOs contained in this food and the partial dose of ready insulin necessary to metabolize the detected CHOs.

According to the invention, the same procedure includes also the computing of an additional insulin dose, relating to the same food, necessary to metabolize the additional CHOs deriving from the delayed transformation of lipids and proteins contained in the eaten food.

Upon completion of the computing step for computing the partial insulin dose (i.e., related to each single food), the management program activates a corresponding step for communicating with the patient. This step includes the visualization of the information relating to the value of such insulin dose that the patient can self-administer directly before or directly after having consumed the food.

The above described step cycle for typological and quantitative evaluation of a food, for computing the partial insulin dose relating to that food and for communicating its value to the patient, is repeated for each food composing the meal.

It is evident that each step of the aforesaid cycle carried out according to the method of the invention, for example the step for computing the partial insulin dose, must be considered substantially a sub-step of the corresponding computing step of the conventional method. According to the invention, the patient is given information relating to an insulin bolus to administer, corresponding to the food he is going to consume.

Since this operation is repeated a number of times, for each food consumed by the patient, he will have to inject more insulin doses for each meal. This is not a great problem, since due to the use of the above mentioned microinfusers, the administration of a predetermined insulin dose means that the patient must only push a button on the microinfuser. Another easy to use system includes a membrane with a needle fixed to the skin, which allows to inject predetermined doses of insulin one after another piercing more times the membrane and not the skin.

According to the method of the invention, the value of the additional insulin dose computed for each food, relating to the part given by the lipids and proteins, is stored by the management program and progressively summed up with the corresponding parts of all the other foods. Consequently, at the end of the meal, the communication procedure is activated again to inform the patient about the additional insulin dose he will have to inject in order to compensate for the transformation of the part of lipids and proteins consumed during the whole meal.

The distribution over time of the total insulin bolus in a plurality of doses to administer during the meal, directly before or after the consumption of each food that composes the meal, allows to simulate the curve of physiological production of endogenous insulin by the pancreas during and immediately after a meal in a much more precise way with respect to the conventional techniques.

However, it is to be considered that according to the nature of the invention various embodiments and versions of the method are possible.

According to the invention, the typology and quantity evaluation steps, for computation of the insulin dose and for the communication with the patient, can be carried out also for groups of foods, not only for a single food, for example, for all starters, for the soups, etc.

According to a further variation, the step for communicating to the patient the insulin dose relating to the single food or to a group of foods takes place immediately after they have been consumed.

According to yet another version of the method, the step for the evaluation of the foods and of the composition of the meal, and the step for the computation of the related partial doses of insulin, are carried out in a single operation before the beginning of the meal, and the steps for communicating different values of such partial doses are carried out, at the patient's request, progressively before the consumption of each food or group of foods, or after a given period of time, defined by the specialist on the basis of the response time to the treatment of the particular subject.

According to another variation of the method, the communication with the patient concerning the additional insulin dose for a food or a group of foods occurs after a given period of time, likewise defined by the specialist. The additional insulin dose for a given food could also be summed up with the main insulin dose of one of the foods consumed afterwards by the patient.

Carrying out of the method for the quantitative definition of the insulin bolus and its distribution over time according to the invention advantageously allows to dose the direct insulin intake by the diabetic patient, necessary to metabolize the CHOs contained in a meal composed in any way, in a manner very similar to the corresponding physiological process in which the pancreas of a healthy subject secretes insulin.

In this way, the above described method allows to considerably limit the oscillations of the patient's blood glucose during and after the consumption of each meal, and thus to prevent possible hyperglycemia or hypoglycemia situations.

The method allows also to define the ways of carrying out the insulin treatment of the diabetic patient taking into consideration all the factors which make the healthy organism secrete insulin during and after a meal, with different distribution over time of the partial doses computed in accordance with the specialist's prescription also on the basis of different origin of the CHOs to metabolize.

It is understood that what above has been described as a pure not limiting example. Therefore, possible changes and different variations of the invention are considered within the protective scope granted to the present technical solution, as described above and claimed below.

## Claims

1. A method for the quantitative definition of the insulin bolus for a diabetic patient, and for the over time management of its administration, said insulin bolus being computed in an interactive electronic terminal, based on one or more computing algorithms stored in said terminal and being operated by means of a management program running on said terminal, based on values of pre-defined physiological parameters of the patient, said parameters being periodically acquired and stored; said method comprising, for each meal that the patient is going to eat:
one or more interactive evaluation steps relating to the type and amount of each course that the patient is going to eat, each course belonging to said meal, until the complete meal has been composed;
a computing step for computing said insulin bolus, said step following said interactive evaluation step, carried out by said management program based on the quantity of carbohydrates contained in the amounts of each of the different chosen courses composing the complete meal;
a communication step, for communicating the amount of said insulin bolus to said patient by means of said interactive electronic terminal; said method being **characterized in that**, for each meal, said computing step for computing the insulin bolus comprises a plurality of subsequent partial computing sub-steps, for computing the partial insulin bolus relating to the amount of carbohydrates contained in said course and, for each one of said partial computing sub-steps, one corresponding communication sub-step, for communicating with said patient, each one of said communication sub-steps comprising the issue, by said management program, of a suggestion containing the value of said partial insulin bolus to be self-administered by said patient together with said course;
**in that**
for each one of said courses composing the meal chosen by the patient, said computing sub-step comprises separate computation of a main insulin bolus, necessary to metabolize the carbohydrate component contained directly in said course, and of an additional insulin bolus, necessary to metabolize the carbohydrate component which will be produced after the food has been eaten, for the transformation of fats and proteins contained in the same course, said computation being made by said management program based on a further algorithm stored in said interactive electronic terminal, information relating to said additional insulin bolus being also provided to the patient in addition to the one relating to the main insulin bolus; and
**in that**
said additional insulin bolus is provided to the patient at the end of his meal, in a single total insulin bolus due for all of the foods consumed during the meal.

2. A method according to Claim 1, **characterized in that** each one of said computing sub-steps for computing the partial insulin bolus, and its corresponding communication with the patient sub-step, are carried out during the corresponding said interactive meal composition evaluation steps, for each courses chosen by the patient.

3. A method according to Claim 1, **characterized in that**, for each one of said courses composing the meal chosen by the patient, said management program provides, on request made by said patient, said corresponding partial communication of the partial insulin bolus to be administered, before said course is eaten.

4. A method according to Claim 1, **characterized in that**, for each one of said courses composing the meal chosen by the patient, said management program provides, on request made by said patient, said corresponding partial communication of the partial insulin bolus to be administered, after said course has been eaten.

5. A method according to Claim 1, **characterized in that** the information relating to said additional insulin bolus is provided to the patient, for each of said courses, in the same partial communication step relating to said main insulin bolus.

6. A method according to Claim 1, **characterized in that** the information relating to said additional insulin bolus is provided to the patient, for each of said courses, at a later time with respect to that of said partial communication step relating to said main insulin bolus.

7. A system for the quantitative definition of the insulin bolus for a diabetic patient, and for the distribution over time of its administration, said insulin bolus being computed based on one or more known computing algorithms, said system comprising an interactive electronic terminal and a management program, which is configured to run on said interactive electronic terminal and is aimed at defining an insulin bolus for a meal that the patient is going to eat;
said management program comprising a meal evaluation procedure aimed at interacting with said patient to define the composition of said meal comprising one or more courses; a computing procedure for defining said insulin bolus based on said computing algorithms, on the type and amount of each course which has been chosen for said meal, and on information relating to physiological parameters of said patient, stored in said interactive electronic terminal;
a communication procedure for communicating said computed insulin bolus to said patient; said system being **characterized in that** said management program comprises means for activating said insulin bolus computing procedure for each of said courses, for computing a partial value of said insulin bolus corresponding to said course, and means for activating said communication procedure for each of said computed values of insulin bolus, said communication comprising said partial value and a suggestion for its administration together with said course; a further algorithm in said management program for composing the meal chosen by the patient for each one of said courses and comprising separate computation of a main insulin bolus, necessary to metabolize the carbohydrate component contained directly in said course, and of an additional insulin bolus, necessary to metabolize the carbohydrate component which will be produced after the food has been eaten, for the transformation of fats and proteins contained in the same course, said computation being made by said management program based on a further algorithm stored in said interactive electronic terminal, information relating to said additional insulin bolus being also provided to the patient in addition to the one relating to the main insulin bolus;
means for communicating said additional insulin bolus provided to the patient at the end of his meal, in a single total insulin bolus due for all of the foods consumed during the meal.

## Patentansprüche

1. Verfahren zur quantitativen Definition des Insulinbolus für einen Diabetespatienten und zur zeitlichen Verwaltung seiner Verabreichung, wobei der Insulinbolus in einem interaktiven elektronischen Terminal berechnet wird, basierend auf einem oder mehreren in dem Terminal gespeicherten Berechnungsalgorithmen und mittels eines auf dem Terminal laufenden Verwaltungsprogramms betrieben werden, das auf Werten vordefinierter physiologischer Parameter des Patienten basiert, wobei die Parameter periodisch erfasst und gespeichert werden; wobei das Verfahren für jede Mahlzeit, die der Patient essen wird, umfasst: einen oder mehrere interaktive Bewertungsschritte in Bezug auf die Art und Menge jedes Gerichte, den der Patient essen wird, wobei jeder Gericht zu dieser Mahlzeit gehört, bis die vollständige Mahlzeit zusammengestellt wurde; einen Berechnungsschritt zum Berechnen des Insulinbolus, wobei der Schritt dem interaktiven Bewertungsschritt folgt, der von dem Managementprogramm auf der Grundlage der Menge an Kohlenhydraten durchgeführt wird, die in den Mengen jedes der verschiedenen ausgewählten Gänge enthalten ist, aus denen die vollständige Mahlzeit besteht; einen Kommunikationsschritt zum Kommunizieren der Menge des Insulinbolus an den Patienten mittels des interaktiven elektronischen Terminals; wobei die Verfarhen **dadurch gekennzeichnet** ist, für jede Mahlzeit umfasst der Berechnungsschritt zum Berechnen des Insulinbolus mehrere nachfolgende Teilberechnungsunterschritte zum Berechnen des Teilinsulinbolus in Bezug auf die Menge an Kohlenhydraten, die in dem Gericht enthalten sind, und für jeden der Teilberechnungsunterschritte Schritte, ein entsprechender Kommunikationsunterschritt zur Kommunikation mit dem Patienten, wobei jeder der Kommunikationsunterschritte durch das Verwaltungsprogramm die Ausgabe eines Vorschlags umfasst, der den Wert des von ihm selbst zu verabreichenden partiellen Insulinbolus enthält Patient zusammen mit besagtem Gericht; darin Für jeden der Gerichte, aus denen sich die vom Patienten gewählte Mahlzeit zusammensetzt, umfasst der Berechnungsunterschritt die getrennte Berechnung eines Hauptinsulinbolus, der zur Metabolisierung der direkt in dem Gerichte enthaltenen Kohlenhydratkomponente erforderlich ist, und eines zusätzlichen Insulinbolus, der zur Metabolisierung erforderlich ist die Kohlenhydratkomponente, die nach dem Verzehr des Lebensmittels für die Umwandlung von Fetten und Proteinen, die im gleichen Verlauf enthalten sind, produziert wird, wobei die Berechnung durch das Verwaltungsprogramm auf der Grundlage eines weiteren Algorithmus durchgeführt wird, der in dem interaktiven elektronischen Terminal gespeichert ist, Informationen in Bezug auf wobei dem Patienten zusätzlich zu demjenigen, der sich auf den Hauptinsulinbolus bezieht, auch zusätzlicher Insulinbolus zur Verfügung gestellt wird; und darin Der zusätzliche Insulinbolus wird dem Patienten am Ende seiner Mahlzeit in einem einzigen Gesamtinsulinbolus zur Verfügung gestellt, der für alle während der Mahlzeit verzehrten Lebensmittel fällig ist.

2. Verfahren nach Anspruch 1, **dadurch gekennzeichnet**, jeder der Berechnungsunterschritte zum Berechnen des partiellen Insulinbolus und seine entsprechende Kommunikation mit dem Patientenunterschritt werden während der entsprechenden der interaktiven Bewertungsschritte für die Zusammensetzung der Mahlzeiten für jeden vom Patienten gewählten Gericht durchgeführt.

3. Verfahren nach Anspruch 1, **dadurch gekennzeichnet**, für jeden der Gerichte, aus denen sich die vom Patienten gewählte Mahlzeit zusammensetzt, stellt das Managementprogramm auf Anfrage des Patienten die entsprechende Teilkommunikation des zu verabreichenden Teilinsulinbolus bereit, bevor der Gericht gegessen wird.

4. Verfahren nach Anspruch 1, **dadurch gekennzeichnet**, für jeden der Gerichte, aus denen sich die vom Patienten gewählte Mahlzeit zusammensetzt, stellt das Managementprogramm auf Anfrage des Patienten die entsprechende Teilkommunikation des zu verabreichenden Teilinsulinbolus nach dem Verzehr des Gerichte bereit.

5. Verfahren nach Anspruch 1, **dadurch gekennzeichnet**, die Informationen, die sich auf den zusätzlichen Insulinbolus beziehen, werden dem Patienten für jeden der Gerichte in demselben Teilkommunikationsschritt bereitgestellt, der sich auf den Hauptinsulinbolus bezieht.

6. Verfahren nach Anspruch 1, **dadurch gekennzeichnet**, die Informationen bezüglich des zusätzlichen Insulinbolus werden dem Patienten für jeden der Gerichte zu einem späteren Zeitpunkt in Bezug auf den Teilkommunikationsschritt bezüglich des Hauptinsulinbolus zur Verfügung gestellt.

7. System zur quantitativen Definition des Insulinbolus für einen Diabetespatienten und zur Verteilung über die Zeit seiner Verabreichung, wobei der Insulinbolus auf der Grundlage eines oder mehrerer bekannter Rechenalgorithmen berechnet wird, wobei das System ein interaktives elektronisches Terminal und ein Verwaltungsprogramm, das so konfiguriert ist, dass es auf dem interaktiven elektronischen Terminal ausgeführt wird und darauf abzielt, einen Insulinbolus für eine Mahlzeit zu definieren, die der Patient essen wird; wobei das Managementprogramm ein Mahlzeitbewertungsverfahren umfasst, das darauf abzielt, mit dem Patienten zu interagieren, um die Zusammensetzung der Mahlzeit zu definieren, die einen oder mehrere Gänge umfasst; ein Berechnungsverfahren zum Definieren des Insulinbolus basierend auf den Berechnungsalgorithmen, auf der Art und Menge jedes Gerichte, der für die Mahlzeit ausgewählt wurde, und auf Informationen bezüglich physiologischer Parameter des Patienten, die in dem interaktiven elektronischen Terminal gespeichert sind; ein Kommunikationsverfahren zum Kommunizieren des berechneten Insulinbolus an den Patienten; Das System ist **dadurch gekennzeichnet, dass** das Verwaltungsprogramm Mittel zum Aktivieren des Insulinbolus-Berechnungsverfahrens für jeden der Gerichte, zum Berechnen eines Teilwerts des Insulinbolus, der dem Gericht entspricht, und Mittel zum Aktivieren des Kommunikationsverfahrens für jeden der berechneten umfasst Werte des Insulinbolus, wobei die Mitteilung den Teilwert und einen Vorschlag für seine Verabreichung zusammen mit dem genannten Gericht umfasst; einen weiteren Algorithmus in dem Managementprogramm zum Zusammenstellen der vom Patienten für jeden der Gerichte gewählten Mahlzeit und umfassendes Berechnen eines Hauptinsulinbolus, der zur Metabolisierung der direkt in dem Gericht enthaltenen Kohlenhydratkomponente erforderlich ist, und eines zusätzlichen Insulinbolus; notwendig, um die Kohlenhydratkomponente, die nach dem Verzehr des Lebensmittels produziert wird, für die Umwandlung von Fetten und Proteinen, die im gleichen Verlauf enthalten sind, zu metabolisieren, wobei die Berechnung durch das Managementprogramm auf der Grundlage eines weiteren Algorithmus durchgeführt wird, der in dem interaktiven elektronischen Terminal gespeichert ist; Informationen in Bezug auf den zusätzlichen Insulinbolus werden dem Patienten zusätzlich zu demjenigen, der sich auf den Hauptinsulinbolus bezieht, ebenfalls zur Verfügung gestellt; Mittel zum Kommunizieren des zusätzlichen Insulinbolus, der dem Patienten am Ende seiner Mahlzeit zur Verfügung gestellt wird, in einem einzigen Gesamtinsulinbolus, der für alle während der Mahlzeit konsumierten Lebensmittel fällig ist.

## Revendications

1. Procédé pour la définition quantitative du bolus d'insuline pour un patient diabétique, et de gestion dans le temps de son administration, ledit bolus d'insuline étant calculé dans un terminal électronique interactif, à partir d'un ou plusieurs algorithmes de calcul stockés dans ledit terminal et fonctionnant au moyen d'un programme de gestion s'exécutant sur ledit terminal, sur la base de valeurs de paramètres physiologiques prédéfinis du patient, lesdits paramètres étant périodiquement acquis et stockés; ledit procédé comprenant, pour chaque repas que le patient va manger:
une ou plusieurs étapes d'évaluation interactive relatives au type et à la quantité de chaque plat que le patient va manger, chaque plat appartenant audit repas, jusqu'à ce que le repas complet ait été composé; une étape de calcul pour calculer ledit bolus d'insuline, ladite étape faisant suite à ladite étape d'évaluation interactive, réalisée par ledit programme de gestion en fonction de la quantité de glucides contenue dans les quantités de chacun des différents plats choisis composant le repas complet; une étape de communication, pour communiquer la quantité dudit bolus d'insuline audit patient au moyen dudit terminal électronique interactif; ledit procédé étant **caractérisé en ce que** pour chaque repas, ladite étape de calcul pour calculer le bolus d'insuline comprend une pluralité de sous-étapes de calcul partielles ultérieures, pour calculer le bolus partiel d'insuline relatif à la quantité de glucides contenue dans ledit plat et, pour chacune desdites sous-étapes de calcul partiel, une sous-étape de communication correspondante, pour communiquer avec ledit patient, chacune desdites sous-étapes de communication comprenant l'émission, par ledit programme de gestion, d'une suggestion contenant la valeur dudit bolus d'insuline partiel à auto-administrer par ledit patient avec ledit plat;
**en ce que**
pour chacun desdits plats composant le repas choisi par le patient, ladite sous-étape de calcul comprend le calcul séparé d'un bolus d'insuline principal, nécessaire pour métaboliser le composant glucidique contenu directement dans ledit plat, et d'un bolus d'insuline supplémentaire, nécessaire pour métaboliser le composant glucidique qui sera produit après la consommation de l'aliment, pour la transformation des graisses et des protéines contenues dans le même plat, ledit calcul étant effectué par ledit programme de gestion sur la base d'un autre algorithme stocké dans ledit terminal électronique interactif, des informations relatives à ledit bolus d'insuline supplémentaire étant également fourni au patient en plus de celui relatif au bolus d'insuline principal; et
**en ce que**
ledit bolus d'insuline supplémentaire est fourni au patient à la fin de son repas, en un seul bolus d'insuline totale dû pour tous les aliments consommés au cours du repas.

2. Procédé selon la Revendication 1, **caractérisé en ce que** chacune desdites sous-étapes de calcul pour calculer le bolus d'insuline partiel, et sa communication correspondante avec la sous-étape du patient, sont effectuées pendant lesdites étapes d'évaluation de composition de repas interactives correspondantes, pour chaque plat choisi par le patient.

3. Procédé selon la Revendication 1, **caractérisé en ce que**, pour chacun desdits plats composant le repas choisi par le patient, ledit programme de prise en charge prévoit, sur demande dudit patient, ladite correspondante communication partielle du bolus d'insuline partiel à administrer, avant que ledit plat ne soit mangé.

4. Procédé selon la Revendication 1, **caractérisé en ce que**, pour chacun desdits plats composant le repas choisi par le patient, ledit programme de prise en charge prévoit, sur demande dudit patient, ladite communication partielle correspondante du bolus d'insuline partiel à administrer, après que ledit plat a été mangé.

5. Procédé selon la Revendication 1, **caractérisé en ce que** les informations relatives audit bolus d'insuline supplémentaire sont fournies au patient, pour chacun desdits plats, dans la même étape de communication partielle relative audit bolus d'insuline principal.

6. Procédé selon la Revendication 1, **caractérisé en ce que** l'information relative audit bolus d'insuline supplémentaire est fournie au patient, pour chacun desdits plats, à un moment ultérieur par rapport à celui de ladite étape de communication partielle relative audit bolus d'insuline principal.

7. Système pour la définition quantitative du bolus d'insuline pour un patient diabétique, et pour la distribution dans le temps de son administration, ledit bolus d'insuline étant calculé à partir d'un ou plusieurs algorithmes de calcul connus, ledit système comprenant un terminal électronique interactif et un programme de gestion, qui est configuré pour s'exécuter sur ledit terminal électronique interactif et vise à définir un bolus d'insuline pour un repas que le patient va manger;
ledit programme de gestion comprenant une procédure d'évaluation de repas visant à interagir avec ledit patient pour définir la composition dudit repas comprenant un ou plusieurs plats; une procédure de calcul pour définir ledit bolus d'insuline à partir desdits algorithmes de calcul, du type et de la quantité de chaque plat qui a été choisi pour ledit repas, et des informations relatives aux paramètres physiologiques dudit patient, stockées dans ledit terminal électronique interactif;
une procédure de communication pour communiquer ledit bolus d'insuline calculé audit patient; ledit système étant **caractérisé en ce que** ledit programme de gestion comprend des moyens pour activer ladite procédure de calcul de bolus d'insuline pour chacun desdits plats, pour calculer une valeur partielle dudit bolus d'insuline correspondant audit plats, et des moyens pour activer ladite procédure de communication pour chacun desdits plats calculés. des valeurs de bolus d'insuline, ladite communication comprenant ladite valeur partielle et une suggestion pour son administration conjointement avec ledit plat; un autre algorithme dans ledit programme de gestion pour composer le repas choisi par le patient pour chacun desdits plats et comprenant le calcul séparé d'un bolus d'insuline principal, nécessaire pour métaboliser le composant glucidique contenu directement dans ledit plat, et d'un bolus d'insuline supplémentaire, nécessaire pour métaboliser le composant glucidique qui sera produit après la consommation de l'aliment, pour la transformation des graisses et des protéines contenues dans le même plat, ledit calcul étant effectué par ledit programme de gestion sur la base d'un autre algorithme stocké dans ledit terminal électronique interactif, des informations relatives audit bolus d'insuline supplémentaire étant également fournies au patient en plus de celle relative au bolus d'insuline principal; des moyens pour communiquer ledit bolus d'insuline supplémentaire fourni au patient à la fin de son repas, en un seul bolus d'insuline totale dû pour tous les aliments consommés au cours du repas.
